# FASCICULE DE BREVET EUROPEEN

(11) **EP 0 538 442 B1**
(45) Date de publication et mention de la délivrance du brevet: **21.06.1995**
(21) Numéro de dépôt: 92910451.1
(22) Date de dépôt: 29.04.1992
(51) Int. Cl.: A61K 9/127, A61K 31/415

(54) **PROCEDE POUR AMELIORER L'EFFICACITE THERAPEUTIQUE D'AGENTS ANTIFONGIQUES LIPOSOLUBLES DE LA FAMILLE DES IMIDAZOLES ET COMPOSITION POUR LA MISE EN OEUVRE DE CE PROCEDE**
VERFAHREN ZUR ERHOEHUNG DER THERAPEUTISCHEN WIRKSAMKEIT FETTLOESLICHER FUNGIZIDEAUS DER FAMILIE DER IMIDAZOLE SOWIE MITTEL ZUR DURCHFUEHRUNG DIESES VERFAHREN
METHOD FOR IMPROVING THE THERAPEUTIC EFFICIENCY OF LIPOSOLUBLE ANTIFUNGAL AGENTS FROM THE IMIDAZOLE FAMILY, AND COMPOSITION FOR IMPLEMENTING SAME

(30) Priorité: 03.05.1991 FR 9105458
(43) Date de publication de la demande: 28.04.1993
(73) Titulaire: L'OREAL, 75008 Paris (FR)
(72) Inventeur: LAUGIER, Jean-Pierre, F-92160 Antony (FR); SEGOT, Evelyne, F-94130 Nogent-sur-Marne (FR); SIMONNET, Jean-Thierry, F-75011 Paris (FR)
(74) Mandataire: Peuscet, Jacques
(86) Numéro de dépôt international: FR9200380
(87) Numéro de publication internationale: WO9219225

(56) Documents cités:
- WO-A-87/06460
- WO-A-89/03679
- WORLD PATENTS INDEX LATEST Week 9038, Derwent Publications Ltd., London, GB; AN 90-287819 (38) & JP-A-2 204 413 (Nonogawa Shoji) 14 August 1990

## Description

La présente invention concerne un procédé pour améliorer l'efficacité thérapeutique d'agents antifongiques de la famille des imidazoles, ainsi qu'une composition pour la mise en oeuvre de ce procédé.

On sait que les imidazoles, tels que l'éconazole et le miconazole, ont une excellente activité antifongique et anti-bactérienne, et sont plus particulièrement préconisés dans le traitement des mycoses cutanées à dermatophytes ou à Candida. Ces antifongiques sont généralement utilisés sous la forme d'émulsions huile-dans-eau ou de dispersions aqueuses de vésicules lipidiques à structure lamellaire.

Il est, en effet, connu que certains lipides sont susceptibles de former, par dispersion dans une phase aqueuse, des vésicules constituées d'un ou plusieurs feuillets lamellaires lipidiques, disposés approximativement de façon concentrique et encapsulant une phase aqueuse. Dans le cas où les vésicules sont préparées à partir de lipides amphiphiles non-ioniques, on obtient "des vésicules non-ioniques" ; dans le cas où l'on utilise des lipides amphiphiles ioniques, on obtient des "liposomes". Les vésicules non-ioniques, ainsi qu'un procédé pour les préparer, sont décrits, par exemple, dans le brevet FR-A-2 315 991. De façon connue, les vésicules encapsulent une phase aqueuse E et sont dispersées dans une phase aqueuse D, les phases aqueuses E et D pouvant contenir des additifs identiques ou différents.

On a déjà proposé d'introduire des agents antifongiques imidazolés dans les feuillets lipidiques de liposomes.

Notamment, dans la demande de brevet EP-A-0 119 020, on a proposé d'associer, entre autres, le kétoconazole, l'isoconazole et le miconazole, avec les lipides devant former des liposomes. La demande de brevet EP-A-0 177 223 décrit une composition pharmaceutique qui comprend un composé biologiquement actif faiblement soluble dans l'eau, tel que l'éconazole nitrate ou l'éconazole base, d'une part encapsulé dans des liposomes multi-lamellaires, d'autre part sous forme d'une solution saturée dudit composé, et enfin sous forme solide, les trois formes dudit composé étant dispersées dans un gel hydrocolloïdal qui permet l'application locale. Ces préparations contiennent le composé actif sous trois formes pour pallier l'instabilité physique des liposomes d'éconazole qui relarguent ledit composé actif dans le véhicule d'administration. Par ailleurs, la demande de brevet EP-A-0 253 619 décrit un procédé de préparation de liposomes bi-couches renfermant un composé biologiquement actif, entre autres l'éconazole, utilisé tel quel ou bien introduit dans un support, notamment un gel ; cependant, la présence de solvant organique, comme l'éthanol, dans la préparation obtenue présente l'inconvénient de déstabiliser les liposomes.

On a également proposé d'introduire des agents fongicides dans les feuillets de vésicules constituées à partir de lipides non-ioniques à une chaîne grasse. Ainsi, la demande internationale WO 88/06 881 décrit la préparation de vésicules lipidiques, dont les feuillets lipidiques sont constitués de lipides non-ioniques à une chaîne grasse ; ces vésicules non-ioniques peuvent renfermer des pesticides, des fongicides et des antiseptiques. De même, la demande internationale WO 88/06 883 décrit un procédé de fabrication de vésicules non-ioniques à partir de lipides non-ioniques à une chaîne grasse pouvant renfermer des substances lipophiles, tels que des fongicides, ou bien des substances hydrophiles.

Les expériences conduites par la Société déposante ont montré que les vésicules non-ioniques, telles que décrites dans les demandes internationales précitées, ne peuvent pas contenir, dans leurs parois lipidiques, 1 % en poids (ou plus) de molécules lipophiles, de la famille des imidazoles telles que l'éconazole, sans qu'il n'y ait fuite du principe actif vers le milieu extérieur, car les parois, constituées de lipides à une seule chaîne grasse sont trop rigides pour incorporer une telle quantité de molécules lipopniles de la famille des imidazoles. Ces vésicules non-ioniques présentent donc l'inconvénient de ne pas incorporer une quantité suffisante d'agent antifongique lipophile de la famille des imidazoles pour permettre une activité thérapeutique optimale.

La société déposante a découvert, de façon tout à fait surprenante, que des vésicules à base de lipides non-ioniques à deux chaînes grasses restent stables et permettent de réaliser des formulations stables en présence d'un agent antifongique imidazolé, si le pH de la phase vésiculaire est compris entre 6 et 8. En effet, lorsque cette condition de pH n'est pas remplie, les vésicules obtenues, contenant l'agent antifongique, ne conduisent pas à une stabilité satisfaisante et ne permettent pas de réaliser un gel ou une crème stable contenant le composé actif totalement encapsulé. Il est connu par la demande de brevet FR-A-89-07947, d'utiliser une base, notamment la triéthanolamine, dans des composition contenant des vésicules constituées à partir de lipides non-ioniques à deux chaînes grasses. Cependant, dans ces compositions, le rôle de la base est de neutraliser le gélifiant utilisé pour gélifier le milieu aqueux de dispersion de la composition. Il a été démontré, conformément à l'exemple 3 ci-après, que si l'on utilise un gel pré-neutralisé dans une composition contenant de l'éconazole encapsulé, l'absence de base conduit à une composition instable.

La présente invention a donc pour objet un procédé pour améliorer l'efficacité thérapeutique des agents antifongiques liposolubles appartenant à la famille des imidazoles, dans lequel on introduit au moins l'un desdits agents antifongiques dans une phase lipidique amphiphile non-ionique susceptible de former, par dispersion dans une phase aqueuse, des vésicules constituées de feuillets lamellaires, et, de façon connue, on forme lesdites vésicules encapsulant une phase aqueuse ; selon l'invention, on choisit, comme lipide(s) amphiphile(s) non-ionique(s), un (ou des) lipide(s) à deux chaînes grasses et on ajuste le pH de la phase vésiculaire, à l'aide d'au moins un agent de neutralisation, à une valeur comprise entre environ 6 et 8.

Le (ou les) agent(s) antifongique(s) de la famille des imidazoles est (sont), de préférence, introduit(s) sous forme de base dans la phase lipidique ; il(s) est (sont) choisi(s) notamment parmi l'éconazole, le miconazole, l'itraconazole, le fluconazole, le terconazole, le clotrimazole, le butoconazole, le tioconazole, le vibunazole, l'oxyconazole, le sulconazole, le bifonazole, le fenticonazole, le méticonazole, le croconazole et le kétoconazole.

Le(s) lipide(s) amphiphile(s) non-ionique(s) constituant les feuillets des vésicules a (ont), de préférence, pour formule : 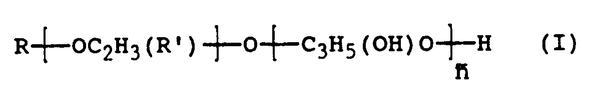
dans laquelle :
· -OC₂H₃(R')- désigne les structures suivantes, prises en mélange ou séparément : 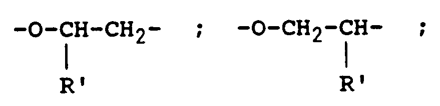 R et R' représentant chacun une chaîne aliphatique, linéaire ou ramifiée, saturée ou insaturée, contenant de 12 à 18 atomes de carbone, ou bien un reste R¹CO où R¹ est un radical aliphatique, linéaire ou ramifié, en C₁₁-C₁₇ ;
· -C₃H₅(OH)O- désigne les structures suivantes, prises en mélange ou séparément : -CH₂-CH(OH)-CH₂-O- ; 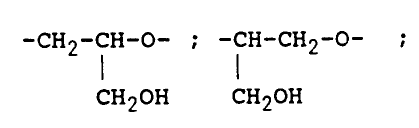 et
· n̅ est une valeur statistique moyenne comprise entre 2 et 6.

De façon connue, on peut associer au(x) lipide(s) amphiphile(s) non-ionique(s) divers additifs en vue de modifier la perméabilité ou la charge superficielle des vésicules. Ces additifs sont notamment :
- des esters phosphoriques d'alcools gras et les sels de sodium correspondants, par exemple, le dihexadécyl ou le ditétradécylphosphate ou les sels de sodium correspondants ;
- les alcools et diols à longue chaîne ;
- les stérols, plus particulièrement, le cholestérol et le β-sitostérol :
- les amines à longue chaîne et leurs dérivés ammonium quaternaires, par exemple, le bromure de dodécyl-diméthylammonium ;
- les bis-hydroxyalkylamines ;
- les amines grasses polyoxyéthylénées et leurs sels ;
- les esters d'amino-alcools à longue chaîne, ainsi que leurs sels et dérivés ammonium quaternaires ;
- les alkylsulfates, par exemple, l'hexadécyl sulfate de sodium ; et
- les dérivés ioniques des stérols, tels que les phosphate et sulfate de cholestérol.

Dans le cadre de la présente invention, les esters phosphoriques d'alcools gras et les sels de sodium correspondants sont particulièrement appropriés ; ces esters phosphoriques peuvent avantageusement être également associés à du cholestérol.

Les additifs associés représentent généralement moins de 10% en poids des lipides amphiphiles non-ioniques.

Le (ou les) agent(s) antifongique(s) de la famille des imidazoles, qui est (ou sont) contenu(s) dans la phase lipidique lamellaire, représente(nt) généralement 1 à 12,5 % en poids des lipides totaux constituant les feuillets des vésicules (y compris les éventuels additifs associés).

Selon l'invention, on ajuste le pH de la phase vésiculaire à une valeur comprise entre 6 et 8, de préférence entre 6,5 et 7,5, à l'aide d'une base organique telle que la mono-, la di- ou la triéthanolamine, la diisopropanolamine, le tris(hydroxyméthyl)aminométhane, la lysine, l'arginine ou la tétra(hydroxypropyl)éthylènediamine, ou à l'aide d'une base minérale telle que l'hydroxyde de sodium ou de potassium.

Les vésicules ont, de préférence, un diamètre moyen compris entre 10 nm et 5 000 nm, et, mieux encore, entre 10 et 1 000 nm.

La présente invention a également pour objet, à titre de produit nouveau, une composition pour la mise en oeuvre du procédé tel que défini ci-dessus, comportant une phase aqueuse D, dans laquelle sont dispersées des vésicules de lipide(s) amphiphile(s) non-ionique(s) à deux chaînes grasses contenant, dans leurs feuillets lipidiques, au moins un agent antifongique de la famille des imidazoles.

Des exemples d'agents antifongiques de la famille des imidazoles, de lipides amphiphiles non-ioniques et de leurs éventuels additifs ont été donnés ci-dessus.

Le (ou les) agent(s) antifongique(s) de la famille des imidazoles introduits dans la phase lipidique lamellaire des vésicules représente(nt) généralement de 0,05 à 1% en poids, et, de préférence, de 0,2 à 0,75% en poids, par rapport au poids total de la composition.

Selon la présente invention, la phase aqueuse D, dans laquelle sont dispersées les vésicules, peut être gélifiée. La phase aqueuse D peut être également une dispersion d'un liquide non-miscible à l'eau dans un milieu aqueux continu, gélifié ou non.

Pour obtenir un gel, on ajoute au milieu aqueux de dispersion un gélifiant. Parmi les gélifiants utilisables, on peut citer les acides polyacryliques réticulés, par exemple, ceux commercialisés sous la dénomination commerciale "CARBOPOL" ou "CARBOMER" par la Société GOODRICH, les dérivés de cellulose, par exemple l'hydroxypropylcellulose, la carboxyméthylcellulose, l'hydroxyéthylcellulose, l'hydroxypropylméthylcellulose, les copolymères acrylamide/ acrylate de sodium, les copolymères vinylpyrrolidone/acétate de vinyle, les dérivés d'algues ou encore des gommes naturelles.

Le gélifiant peut être introduit en des quantités comprises entre 0,1 et 2% en poids par rapport au poids total de la composition.

Il est connu par les brevets FR-B-2 485 921 et 2 490 504 que les vésicules de lipide(s) amphiphile(s) non-ionique(s) stabilisent des dispersions en phase aqueuse de liquides non-miscibles à l'eau, sans qu'il soit nécessaire d'ajouter un agent émulsifiant. On peut utiliser cette propriété en ajoutant à la composition de l'invention un liquide non-miscible au milieu aqueux dans lequel sont dispersées les vésicules.

Le liquide non-miscible à l'eau est, de préférence, une huile. L'huile utilisée est avantageusement prise dans le groupe formé par les esters d'acides gras et de polyols, et les esters d'acides gras et d'alcools ramifiés de formule R²-COOR³, dans laquelle R² représente un reste d'un acide gras supérieur en C₈-C₂₀ et R³ représente une chaîne hydrocarbonée ramifiée en C₃-C₂₀. Si l'huile est un ester d'acide gras et de polyol, on préfère qu'elle soit choisie dans le groupe formé par les huiles de tournesol, de maïs, de soja, de courge, de pépins de raisin, de sésame, de macadamia, de bourrache, et de cassis, et par le tricaprocaprylate de glycérol, et les triglycérides d'acide caprique/caprylique. Si l'huile est un ester d'acide gras supérieur et d'alcool ramifié, on préfère que ce soit l'huile de Purcellin. D'autres huiles végétales peuvent aussi être utilisées, telle que l'huile de jojoba.

Selon l'invention, le liquide non-miscible à l'eau peut également être un hydrocarbure ou un polysiloxane. L'hydrocarbure est, par exemple, de l'huile de vaseline, l'huile de paraffine, le perhydrosqualène. Le polysiloxane est, par exemple, la cyclométhicone, le diméthylpolysiloxane ou le phénylpolysiloxane.

La quantité de liquide non-miscible à l'eau s'élève généralement au plus à 50 % en poids par rapport au poids total de la composition.

On peut introduire, dans la phase aqueuse D, d'autres additifs hydrosolubles pharmaceutiquement acceptables : ils sont, de préférence, pris dans le groupe formé par les bactéricides, les antioxydants, les conservateurs, les agents chélatants, les opacifiants ou les colorants.

Les additifs hydrosolubles utilisables dans la phase aqueuse D peuvent également être ajoutés à la phase aqueuse E, qui est encapsulée dans les vésicules, les additifs contenus dans les phares E et D étant identiques ou différents.

En outre, dans le cas où la phase aqueuse D est une dispersion dans un milieu aqueux d'un liquide non-miscible à l'eau, ledit liquide peut également contenir au moins un additif liposoluble pharmaceutiquement acceptable.

Les feuillets lipidiques peuvent également contenir, de façon connue, au moins un additif liposoluble pharmaceutiquement actif. Ces additifs liposolubles pharmaceutiquement acceptables sont, de préférence pris dans le groupe formé par les antioxydants, tel que le butylhydroxy anisole, le butylhydroxytoluène, le gallate de propyle, les conservateurs, tels que les esters de l'acide parahydroxybenzoïque, le 5 chloro 2-(2,4-dichloro-phenoxy)phénol, les émollients, tels que les alcools gras en C₈-C₂₀, les cires microcristallines. Les compositions selon l'invention peuvent être appliquées sur la peau pour le traitement des mycoses cutanées à dermatophytes ou à Candida ou peuvent être appliquées sur l'ongle pour le traitement des périonyxis (mycose sur le contour de l'ongle).

Les compositions selon l'invention peuvent être préparées par tout procédé connu de fabrication de vésicules de lipide(s) non-ionique(s). L'agent antifongique est ajouté au mélange lipidique avant d'effectuer l'hydratation dudit mélange lipidique et de former les vésicules.

On peut par exemple utiliser le procédé suivant. On mélange le lipide amphiphile non-ionique et éventuellement, l'additif associé, l'agent antifongique et l'agent de neutralisation, a une température au moins égale à la température de fusion du mélange ; on met le mélange obtenu en contact avec la phase aqueuse à encapsuler et on agite de façon à obtenir une phase lamellaire ; on ajoute ensuite une phase aqueuse de dispersion que l'on agite de façon à obtenir les vésicules. Dans ce procédé, on utilise comme agent de neutralisation une base organique.

On peut également mélanger le lipide amphiphile non-ionique, l'additif associé et l'agent antifongique à une température au moins égale à la température de fusion du mélange ; on met le mélange obtenu en contact avec la phase aqueuse à encapsuler et on agite de façon à obtenir une phase lamellaire ; on ajoute ensuite la phase aqueuse de dispersion que l'on agite de façon à obtenir les vésicules ; puis on neutralise avec l'agent de neutralisation, qui peut être soit une base organique, soit une base minérale.

Lorsqu'on ajoute un agent gélifiant ou un liquide non-miscible à l'eau, ces derniers sont ajoutés à la phase aqueuse de dispersion comme décrit dans le brevet FR-B-2 485 921.

On peut également utiliser le procédé décrit dans le brevet US-A-4 772 471. Selon ce procédé, on dissout dans un solvant le lipide amphiphile non-ionique, et, éventuellement, l'additif associé, l'agent antifongique et l'agent de neutralisation ; on utilise comme agent de neutralisation une base organique ; on évapore le solvant dans un évaporateur rotatif ; on met en contact le film lipidique obtenu avec une phase aqueuse ; et on disperse sous agitation pour obtenir les vésicules dispersées dans une phase aqueuse. Le gel et éventuellement l'huile sont ajoutés, sous agitation, après le stade de dispersion.

Dans une autre variante de ce procédé, l'agent de neutralisation est dissous dans la phase aqueuse et peut être soit une base organique, soit une base minérale.

Pour mieux faire comprendre l'objet de l'invention, on va en décrire maintenant, à titre d'exemples purement illustratifs et non-limitatifs, plusieurs modes de mise en oeuvre.

Les lipides amphiphiles non-ioniques utilisés dans les exemples répondent à la formule (I) dans laquelle R = C₁₂H₂₅ et R' représente un mélange équimolaire des radicaux tétradécyle et hexadécyle.

### EXEMPLE I : Etude comparative de la cinétique de pénétration dans la peau de l'éconazole d'après le type de formulation utilisé.

### (a) Préparation des formulations

On a préparé une émulsion classique huile-dans-eau (formulation A), une dispersion aqueuse de liposomes (formulation B) et une dispersion aqueuse de vésicules lipidiques non-ioniques selon l'invention (formulation C), incorporant chacune de l'éconazole base et ayant la composition ci-après. On a utilisé, dans ces formulations, de l'éconazole base marqué au tritium (éconazole base alcohyl 3H vendu par la société AMERSHAM).

### Formulation A : Emulsion huile-dans-eau

On réalise la formulation suivante :

| | |
|---|---|
| - Mélange de stéarate de polyéthylèneglycol et de stéarate de glycol, vendu sous la dénomination "TEFOSE 63" par la Société GATTEFOSSE | 20,00 g |
| - Mélange d'huile de noyau d'abricot et de polyéthylèneglycol, vendu sous la dénomination "LABRAFIL M 1944 CS" par la Société GATTEFOSSE | 3,00 g |
| - Huile de vaseline | 15,00 g |
| - Butylhydroxyanisole | 0,05 g |
| - Acide benzoïque | 0,20 g |
| - Acide polyacrylique réticulé, vendu sous la dénomination "CARBOMER 940" par la Société GOODRICH | 0,60 g |
| - Parahydroxybenzoate de méthyle | 0,05 g |
| - Triéthanolamine | 0,40 g |
| - Econazole base | 1,00 g |
| - Eau purifiée | 59,7 g |

On procède de la façon suivante :

On mélange à 80°C le "TEFOSE 63", le "LABRAFIL M1944CS", l'huile de vaseline, le butylhydroxy anisole et l'éconazole base jusqu'à obtention d'une phase homogène.

Indépendamment, on solubilise à 95°C dans l'eau, le parahydroxybenzoate de méthyle, l'acide benzoïque et on disperse le "CARBOMER 940". On neutralise cette phase aqueuse par la triéthanolamine. On refroidit à 85°C. Sous vive agitation, on verse la phase aqueuse dans la phase huileuse puis on refroidit progressivement à température ambiante sous agitation lente.

### Formulation B : Préparation de liposomes

On réalise la formulation suivante :

| | |
|---|---|
| - Lécithine de soja non-hydrogénée, vendue sous la dénomination "EPIKURON 145 V" par le Société LUCAS-MEYER | 10,0 g |
| - Butylhydroxytoluène | 0,05 g |
| - α-Tocophérol | 0,10 g |
| - Parahydroxybenzoate de méthyle | 1,00 g |
| - Huile de vaseline | 14,00 g |
| - Alcool cétylique | 1,50 g |
| - Copolymère acrylamide/acrylate de sodium, vendu sous la dénomination "HOSTACERIN PN 73" par la Société HOECHST | 0,30 g |
| - Econazole base | 1,00 g |
| - Eau purifiée qsp | 100 g |

On procède de la façon suivante :
On solubilise l'"EPIKURON 145 V", le butylhydroxytoluène, l'α-Tocophérol et l'éconazole base dans 50 g de dichloro-méthane. On évapore le solvant afin d'obtenir un film lipidique qui est ensuite hydraté par 54,35 g d'eau purifiée additionnée de parahydroxybenzoate de méthyle. On homogénéise à l'aide d'un homogénéisateur haute pression. On disperse dans ce milieu l'huile de vaseline ainsi que l'alcool cétylique dispersé dans l'huile de vaseline. On, introduit ensuite sous faible agitation un gel obtenu par dispersion de l'"HOSTACERIN" PN 73" dans 15,7 g d'eau.

La taille moyenne des liposomes est de 40 nm.

### Formulation C : Préparation de vésicules non-ioniques (selon l'invention)

### Première étape : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 5,5 | 9,00 g |
| - Ditétradécylphosphate | 1,00 g |
| - Econazole base | 1,00 g |

On réalise par fusion à 100-110°C le mélange ayant la formulation ci-dessus, et, on introduit progressivement 57,5 g d'eau purifiée, portée à 80°C, sous agitation lente ; le pH natif mesuré à ce stade est de 4,5 ; après quoi, on agite vivement, dans une turbine munie de pales, jusqu'à l'obtention d'une masse gélifiée blanc cassé.

On maintient la température et l'agitation pendant 10 minutes. On affine le mélange obtenu par passage dans un homogénéiseur haute pression à température ambiante. On neutralise la dispersion vésiculaire à pH 7,2 par 0,4 g de triéthanolamine.

Les vésicules ont un diamètre moyen de 100 nm.

### Deuxième étape : Préparation de la crème

On réalise la formulation suivante :

| | |
|---|---|
| - Dispersion aqueuse de vésicules obtenue à la première étape | 68,9 g |
| - Parahydroxybenzoate de méthyle | 0,10 g |
| - Acide polyacrylique réticulé, vendu sous la dénomination "CARBOMER 940" par la Société GOODRICH (agent gélifiant) | 0,60 g |
| - Huile de vaseline | 15,00 g |
| - Eau purifiée qsp | 100 g |

A la dispersion aqueuse des vésicules obtenue à la première étape on ajoute l'huile de vaseline et on la disperse. On épaissit en dernier lieu le mélange dans une turbine à pales, en lui ajoutant un gel aqueux bien homogène consitué par l'agent gélifiant dissous dans l'eau renfermant le conservateur.

### (b) Protocole de l'étude

Dans une cellule de diffusion dynamique thermostatée du type cellule de Franz telle que décrite dans "The Journal of Investigative Dermatology, volume 64 (1975), pages 190-195", ayant une surface de 1 cm² et un volume de 3 ml, on dispose un dermatome de peau humaine de 380 »m d'épaisseur.

La composition du liquide récepteur est la suivante : solution aqueuse de NaCl à 0,9 % en poids additionnée d'un tensio-actif vendu sous la dénomination "Brij 98" par la société ICI (ether de polyéthylène glycol de l'alcool oléïque de formule CH₃(CH₂)₇CH = CH(CH₂)₇CH₂-(OCH₂CH₂)ₙOH où n est une valeur moyenne égale à 20) à 4 % en poids. Le débit du liquide récepteur est de 6 ml/h. Le nombre d'échantillons par formulation est de 6 et un prélèvement est effectué toutes les heures pendant 24 heures. Les mesures sont traitées par un test de Student.

### (c) Résultats

Les résultats sont rapportés dans le Tableau I ci-après :

**TABLEAU I**

| | Pourcentage d'éconazole base ayant pénétré | Vitesse de pénétration de l'éconazole base (»g/cm² de peau/h) |
|---|---|---|
| Formulation A | 0,105 | 2,57 |
| Formulation B | 0,084 | 2,72 |
| Formulation C | 0,162 | 4,11 |

On constate que la formulation C permet la pénétration d'une plus grande quantité d'éconazole base, ainsi qu'une plus grande vitesse de pénétration. Le fait de véhiculer l'éconazole base dans des vésicules non-ioniques permet donc d'obtenir une meilleure biodisponibilité que lorsque l'on utilise des liposomes classiques ou une émulsion huile-dans-eau, ce qui se traduit par une guérison plus rapide des mycoses cutanées.

### EXEMPLE 2 : Conditions de pH pour la stabilité des vésicules lipidiques non-ioniques selon l'invention.

On a mesuré le pH natif de différentes phases vésiculaires à base du lipide amphiphile non-ionique de formule (I) dans laquelle n̅ est égal à 5,5

Les résultats sont rapportés dans le Tableau II.

**TABLEAU II**

| Phase vésiculaire | n° 1 | n° 2 Invention |
|---|---|---|
| Lipide amphiphile non-ionique | 9 g | 9 g |
| Econazole base | 1 g | 1 g |
| Ditétradécylphosphate | 1 g | 1 g |
| Triéthanolamine | 0 | 0,4g |
| pH | 4,5 | 7,2 |

On a par ailleurs observé ce qui suit :
Les dispersions vésiculaires obtenues avec la phase vésiculaire n° 1 sont instables. On note une coalescence nes vésicules, qui se traduit par des fuites de l'éconazole base et une recristallisation de celui-ci dans la phase externe.

L'instabilité physique des gels est retardée dans le temps, alors que pour une crème, le phénomène se produit immédiatement à t = 0.

Les seules dispersions stables sont celles obtenues avec la phase vésiculaire n° 2 dont le pH est voisin de 7.

### EXEMPLE 3 : Essai comparatif démontrant la présence nécessaire d'une base pour la stabilité des vésicules non-ioniques.

On prépare la formulation D (selon l'invention) suivante :

### Première étape : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 5,5 | 9 g |
| - Ditétradécylphosphate | 1 g |
| - Econazole base | 1 g |

On fond, en agitant doucement, à une température de 100°C-110°C, le mélange ayant la formulation ci-dessus, et, dans le mélange fondu, on introduit progressivement 54,8g d'eau purifiée, portée à 95°C, sous agitation lente : le pH du mélange obtenu est égal à 4,5;pour effectuer la dispersion au cours de laquelle se forme les vésicules, on agite alors vivement ce mélange, dans une turbine munie de pales, jusqu'à l'obtension d'une masse laiteuse blanc cassé.

On maintient la température et l'agitation pendant 10 minutes.

On affine le mélange obtenu par passage dans un homogéneiseur haute pression à température ambiante. On neutralise le mélange à pH7 à l'aide de 0,2 g de triéthanolamine.

Les vésicules ont un diamètre moyen de 100 nm.

### Deuxième étape : Préparation de la crème

On réalise la formulation suivante :

| | |
|---|---|
| - Dispersion aqueuse de vésicules obtenue à la première étape | 6,6g |
| - Copolymère acrylamide/acrylate de sodium, vendu sous la dénomination "HOSTACERIN PN 73" par la Société HOECHST (agent gélifiant pré-neutralisé) | 0,4 g |
| - Huile de vaseline | 15 g |
| - Cyclométhicone, vendue sous la dénomination de "SILICONE 344 FLUIDE" par la Société DOW CORNING | 3 g |
| - Eau purifiée q.s.p. | 100 g |

A la dispersion aqueuse de vésicules obtenue à la première étape, on ajoute la cyclométhicone et le perhydrosqualène sous agitation. On épaissit en dernier lieu le mélange dans une turbine à pales, en lui ajoutant un gel aqueux bien homogène constitué par l'agent gélifiant dissous dans l'eau.

On obtient une crème blanche, lisse, brillante et fine, sous forme d'une dispersion d'huile très fine. Cette composition est parfaitement stable dans des conditions sévères de conservation.

A des fins de comparaison, on prépare une formulation E, identique à la formulation D sauf qu'on ne neutralise pas à pH 7 par la triéthanolamine la phase vésiculaire (lipide amphiphile non-ionique + ditétradécylphosphate + éconazole base + eau) telle qu'obtenue avant la dispersion formatrice des vésicules.

On observe rapidement un relargage d'huile. La dispersion de vésicules est instable et conduit à la formation de deux phases.

### EXEMPLE 4 : Gel

### Première étape : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 5,5 | 9 g |
| - Ditétradécylphosphate | 1 g |
| - Econazole base | 1 g |

On fond, en agitant doucement, à une température de 100°C-110°C, le mélange ayant la formulation ci-dessus, et, dans le mélange fondu, on introduit progressivement 58,6 g d'eau purifiée, portée à 80°C, sous agitation lente : le pH est de 4,5; après quoi on agite vivement, dans une turbine munie de pales, jusqu'à l'obtention d'une masse liquide blanc cassé.

On maintient la température et l'agitation pendant 10 minutes. On affine le mélange obtenu par passage dans un homogénéiseur haute pression à température ambiante. Puis on neutralise par 1 g de triéthanolamine.

Les vésicules ont un diamètre moyen de 100 nm.

### Deuxième étage : Préparation du gel

On réalise la formulation suivante :

| | |
|---|---|
| - Dispersion aqueuse de vésicules obtenue à la première étape | 70 g |
| - Copolymère acrylamide/acrylate de sodium, vendu sous la dénomination "HOSTACERIN PN 73" par la Société HOECHST (agent gélifiant) | 0,4 g |
| - Eau purifiée q.s.p. | 100 g |

On épaissit la dispersion aqueuse de vésicules, dans une turbine à pales, en lui ajoutant un gel aqueux bien homogène constitué par l'agent gélifiant dissous dans l'eau purifiée.

On utilise ce gel à raison de deux applications topiques par jour ( 20 mg/cm² de peau) pour le traitement d'un sujet atteint d'une mycose cutanée pendant une durée de 20 jours, et l'on constate une amélioration significative de l'état des zones traitées.

### EXEMPLE 5 : Gel

### Première étape : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 5,5 | 10 g |
| - Ditétradécylphosphate | 1 g |
| - Cholestérol | 0,5 g |
| - Econazole base | 1 g |
| - Tétrahydroxypropyléthylènediamine, vendue sous la dénomination "QUADROL L" par la Société BASF | 0,3 g |

On fond, en agitant doucement, à une température de 100°C-110°C, le mélange ayant la formulation ci-dessus, et, dans le mélange fondu, on introduit progressivement 57,2 g d'eau purifiée, portée à 95°C, sous agitation lente : le pH est de 7,1; après quoi on agite vivement, dans une turbine munie de pales, jusqu'à l'obtention d'une masse gélifiée blanc cassé.

On maintient la température et l'agitation pendant 10 minutes. On affine le mélange obtenu par passage dans un homogénéiseur haute pression.

Les vésicules ont un diamètre moyen de 100 nm.

### Deuxième étape : Préparation du gel

On réalise la formulation suivante :

| | |
|---|---|
| - Dispersion aqueuse de vésicules obtenue à la première étape | 70 g |
| - Acide polyacrylique réticulé, vendu sous la dénomination "CARBOMER 940" par la Société GOODRICH (agent gélifiant) | 0,4 g |
| - Triéthanolamine | 0,2 g |
| - Parahydroxybenzoate de méthyle | 0,1 g |
| - Eau purifiée q.s.p. | 100 g |

On épaissit la dispersion aqueuse de vésicules, dans une turbine à pales, en lui ajoutant un gel aqueux bien homogène constitué par l'agent gélifiant dissous avec la triéthanolamine dans l'eau renfermant le parahydroxybenzoate de méthyle.

On utilise ce gel à raison de deux applications topiques par jour ( 20 mg/cm² de peau) pour le traitement d'un sujet atteint d'une mycose cutanée pendant une durée de 15 jours, et l'on constate une amélioration significative de l'état des zones traitées.

### EXEMPLE 6 : Crème

### Première étape : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 6 | 10,00 g |
| - Ditétradécylphosphate | 1,00 g |
| - Econazole base | 1,00 g |

On fond, en agitant doucement, à une température de 100°C-110°C, le mélange ayant la formulation ci-dessus, et, dans le mélange fondu, on introduit progressivement 53,7 g d'eau purifiée, portée à 80°C, sous agitation lente : le pH est de 4,5 ; après quoi on agite vivement, dans une turbine munie de pales, jusqu'à l'obtention d'une masse gélifiée blanc cassé.

On maintient la température et l'agitation pendant 10 minutes. On affine le mélange obtenu par passage dans un homogénéiseur haute pression. On neutralise par 0,2 g de monoéthanolamine à pH7.

Les vésicules ont un diamètre moyen de 100 nm.

### Deuxième étape : Préparation de la crème

On réalise la formulation suivante :

| | |
|---|---|
| - Dispersion aqueuse de vésicules obtenue à la première étape | 65,9 g |
| - Parahydroxybenzoate de méthyle | 0,1 g |
| - Acide polyacrylique réticulé, vendu sous la dénomination "CARBOMER 940" par la Société GOODRICH (agent gélifiant) | 0,4 g |
| - Huile de vaseline | 15 g |
| - Cyclométhicone, vendue sous la dénomination de "SILICONE 344 FLUIDE" par la Société DOW CORNING | 3 g |
| - Eau purifiée q.s.p. | 100 g |

A la dispersion aqueuse de vésicules, obtenue à la première étape, on ajoute l'huile de vaseline et la cyclométhicone et on affine le mélange obtenu. On épaissit en dernier lieu le mélange dans une turbine à pales, en lui ajoutant un gel aqueux bien homogéne constitué par l'agent gélifiant dissous dans l'eau renfermant le conservateur.

On utilise cette crème à raison de deux applications topiques par jour ( 20 mg/cm² de peau) pour le traitement d'un sujet atteint d'une mycose cutanée pendant une durée de 16 jours, et l'on constate une amélioration significative de l'état des zones traitées.

### EXEMPLE 7 : Crème

### Première étape : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 5,5 | 9,00 g |
| - Ditétradécylphosphate | 0,9 g |
| - Econazole base | 1,00 g |
| - Cholestérol | 0,1 g |

On fond, en agitant doucement, à une température de 100°C-110°C, le mélange ayant la formulation ci-dessus, et, dans le mélange fondu, on introduit progressivement 52,7 g d'eau purifiée, portée à 80°C, sous agitation lente : le pH est de 4,7 ; après quoi on agite vivement, dans une turbine munie de pales, jusqu'à l'obtention d'une masse laiteuse blanc cassé.

On maintient la température et l'agitation pendant 10 minutes. On affine le mélange obtenu par passage dans un homogénéiseur haute pression à température ambiante. On neutralise à pH 6,8 par 0,2 g de triéthanolamine.

Les vésicules ont un diamètre moyen de 100 nm.

### Deuxième étape : Préparation de la crème

On réalise la formulation suivante :

| | |
|---|---|
| - Dispersion aqueuse de vésicules obtenue à la première étape | 63,9 g |
| - Parahydroxybenzoate de méthyle | 0,1 g |
| - Copolymère acrylamide/acrylate de sodium, vendu sous la dénomination "HOSTACERIN PN 73" par la Société HOECHST (agent gélifiant) | 0,4 g |
| - Triglycérides d'acide caprique/caprylique, vendus sous la dénomination "MIGLYOL" 812" par la Société DYNAMIT NOBEL | 18 g |
| - Perhydrosqualène | 2 g |
| - Eau purifiée qsp | 100 g |

A la dispersion aqueuse de vésicules obtenue à la première étape, on ajoute les triglycérides et le perhydrosqualène et on affine le mélange obtenu. On épaissit en dernier lieu le mélange dans une turbine à pales, en lui ajoutant un gel aqueux bien homogène constitué par l'agent gélifiant dissous dans l'eau renfermant le conservateur.

On utilise dette crème à raison de deux applications topiques par jour ( 20 mg/cm² de peau) pour le traitement d'un sujet atteint d'une mycose cutanée pendant une durée de 15 jours, et l'on constate une amélioration significative de l'état des zones traitées.

### EXEMPLE 8 : Crème

### Première étage : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 6 | 9,00 g |
| - Ditétradécylphosphate | 0,9 g |
| - Econazole base | 1,00 g |

On fond, en agitant doucement, à une température de 100°C-110°C, le mélange ayant la formulation ci-dessus, et, dans le mélange fondu, on introduit progressivement 52,8 g d'eau purifiée, portée à 80°C, sous agitation lente : le pH est de 4,7; après quoi on agite vivement, dans une turbine munie de pales, jusqu'à l'obtention d'une masse laiteuse blanc cassé.

On maintient la température et l'agitation pendant 10 minutes. On affine le mélange obtenu par passage dans un homogénéiseur haute pression. On neutralise à pH 6,8 par 0,2 g de triéthanolamine.

Les vésicules ont un diamètre moven de 100 nm.

### Deuxième étape : Préparation de la crème

On réalise la formulation suivante :

| | |
|---|---|
| - Dispersion aqueuse de vésicules obtenue à la première étape | 64 g |
| - Parahydroxybenzoate de méthyle | 0,1 g |
| - Hydroxyéthylcellulose, vendue sous la dénomination "HHR 250" par la Société "AQUALON" (agent gélifiant) | 0,4 g |
| - Triglycérides d'acide caprique/caprylique, vendus sous la dénomination "MIGLYOL 812" par la Société DYNAMIT NOBEL | 18 g |
| - Perhydrosqualène | 2 g |
| - Eau purifiée q.s.p. | 100 g |

A la dispersion aqueuse de vésicules obtenue à la première étape, on ajoute les triglycérides et le perhydrosqualène et on affine le mélange obtenu. On épaissit en dernier lieu le mélange dans une turbine à pales, en lui ajoutant un gel aqueux bien homogène constitué par l'agent gélifiant dissous dans l'eau renfermant le conservateur.

On utilise cette crème à raison de deux applications topiques par jour ( 20 mg/cm² de peau) pour le traitement d'un sujet atteint d'une mycose cutanée pendant une durée de 15 jours, et l'on constate une amélioration significative de l'état des zones traitées.

### EXEMPLE 9 : Crème

### Première étape : Préparation d'une dispersion aqueuse de vésicules lipidiques non-ioniques

On utilise une phase lipidique formulée comme suit :

| | |
|---|---|
| - Lipide amphiphile non-ionique de formule (I) dans laquelle n̅ est égal à 5,5 | 10,00 g |
| - Ditétradécylphosphate | 0,9 g |
| - Econazole base | 1,00 g |
| - Cholestérol | 0,5 g |

On fond, en agitant doucement, à une température de 100°C-110°C, le mélange ayant la formulation ci-dessus, et, dans le mélange fondu, on introduit progressivement 55,9 g d'eau purifiée, portée à 95°C, sous agitation lente : le pH est de 4,7; après quoi on agite vivement, dans une turbine munie de pales, jusqu'à l'obtention d'une masse laiteuse blanc cassé.

On maintient la température et l'agitation pendant 10 minutes. On affine le mélange obtenu par passage dans un homogénéiseur haute pression. On neutralise à pH 6,9 par 0,2 g de triéthanolamine.

Les vésicules ont un diamètre moyen de 100 nm.

### Deuxième étape : Préparation de la crème

On réalise la formulation suivante :

| | |
|---|---|
| - Dispersion aqueuse de vésicules obtenue à la première étape | 68,5 g |
| - Parahydroxybenzoate de méthyle | 0,5 g |
| - Hydroxypropylcellulose, vendue sous la dénomination "KLUCEL HF" par la Société "AQUALON" (agent gélifiant) | 0,4 g |
| - Cyclométhicone, vendue sous la dénomination de "SILICONE 344 FLUIDE" par la Société DOW CORNING | 5 g |
| - Perhydrosqualène | 10 g |
| - Eau purifiée q.s.p. | 100 g |

A la dispersion aqueuse de vésicule obtenue à la première étape, on ajoute la cyclométhicone et le perhydrosqualène et on affine le mélange obtenu par passage dans un homogénéiseur haute pression. On épaissit en dernier lieu le mélange dans une turbine à pales, en lui ajoutant un gel aqueux bien homogène constitué par l'agent gélifiant dissous dans l'eau renfermant le conservateur.

On utilise cette crème à raison de deux applications topiques par jour (20 mg/cm² de peau) pour le traitement d'un sujet atteint d'une mycose cutanée pendant une durée de 15 jours, et l'on constate une amélioration significative de l'état des zones à traiter.

## Revendications

1. Procédé pour améliorer l'efficacité thérapeutique des agents antifongiques liposolubles appartenant à la famille des imidazoles, dans lequel on introduit au moins l'un desdits agents antifongiques dans une phase lipidique amphiphile non-ionique susceptible de former, par dispersion dans une phase aqueuse, des vésicules constituées de feuillets lamellaires, et, de façon connue, on forme lesdites vésicules encapsulant une phase aqueuse, caractérisé par le fait qu'on choisit, comme lipide(s) amphiphile(s) non-ionique(s), un (ou des) lipide(s) à deux chaînes grasses et on ajuste le pH de la phase vésiculaire, à l'aide d'au moins un agent de neutralisation à une valeur comprise entre 6 et 8.

2. Procédé selon la revendication 1, caractérisé par le fait que le(s) agent(s) antifongique(s) est (sont) introduit(s) dans la phase lipidique sous forme de base.

3. Procédé selon l'une des revendications 1 ou 2, caractérisé par le fait que le(s) agent(s) antifongique(s) imidazolé(s) est (sont) choisi(s) dans le groupe formé par l'éconazole, le miconazole, l'itraconazole, le fluconazole, le terconazole, le clotrimazole, le butoconazole, le tioconazole, le vibunazole, l'oxyconazole, le sulconazole, le bifonazole, le fenticonazole, le méticonazole, le croconazole et le kétoconazole.

4. Procédé selon l'une des revendications 1 à 3, caractérisé par le fait que le(s) lipide(s) amphiphile(s) non-ionique(s) constituant les feuillets des vésicules a (ou ont) pour formule : 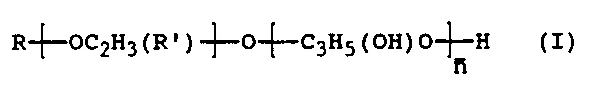 dans laquelle :
· -OC₂H₃(R')- désigne les structures suivantes, prises en mélange ou séparément : 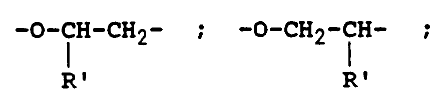 R et R' représentant chacun une chaîne aliphatique, linéaire ou ramifiée, saturée ou insaturée, contenant de 12 à 18 atomes de carbone, ou bien un reste R¹CO où R¹ est un radical aliphatique, linéaire ou ramifié, en C₁₁-C₁₇ ;
· -C₃H₅(OH)O- désigne les structures suivantes, prises en mélange ou séparément :
-CH₂-CH(OH)-CH₂-O- ; 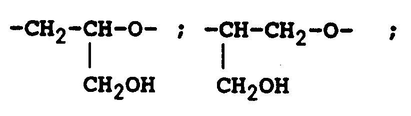 et
· n̅ est une valeur statistique moyenne comprise entre 2 et 6.

5. Procédé selon l'une des revendications 1 à 4, caractérisé par le fait qu'au(x) lipide(s) amphiphile(s) non-ionique(s) est associé au moins un additif destiné à modifier la perméabilité ou la charge superficielle des vésicules et choisi dans le groupe formé par les esters phosphoriques d'alcools gras et les sels de sodium correspondants, les alcools et diols à longue chaîne, les stérols, les amines à longue chaîne et leurs dérivés ammonium quaternaires, les bis-hydroxyalkylamines, les amines grasses polyoxyéthylénées et leurs sels, les esters d'amino-alcools à longue chaîne et leurs sels et dérivés ammonium quaternaires, les alkylsulfates et les dérivés ioniques des stérols.

6. Procédé selon l'une des revendications 1 à 5, caractérisé par le fait que le (ou les) agent(s) antifongique(s) de la famille des imidazoles, qui est (ou sont) contenu(s) dans la phase lipidique lamellaire, représente(nt) 1 à 12,5% en poids par rapport aux lipides totaux constituant les feuillets des vésicules.

7. Procédé selon l'une des revendications 1 à 6, caractérisé par le fait que l'on ajuste le pH de la phase vésiculaire à une valeur comprise entre 6,5 et 7,5.

8. Procédé selon l'une des revendications 1 à 7, caractérisé par le fait qu'on ajuste le pH de la phase vésiculaire à l'aide d'au moins une base choisie dans le groupe formé par la mono-, la di- ou la triéthanolamine, la diisopropanolamine, le tris(hydroxyméthyl)aminométhane, la lysine, l'arginine, la tétra(hydroxypropyl)éthylènediamine l'hydroxyde de sodium et l'hydroxyde de potassium.

9. Procédé selon l'une des revendications 1 à 8, caractérisé par le fait que les vésicules ont un diamètre moyen compris entre 10 nm et 5 000 nm.

10. Composition pour la mise en oeuvre du procédé tel que défini à l'une des revendications 1 à 3, caractérisée par le fait qu'elle comporte une phase aqueuse D dans laquelle sont dispersées des vésicules de lipide(s) amphiphile(s) non-ionique(s) à deux chaînes grasses contenant au moins un agent antifongique choisi dans la famille des imidazoles.

11. Composition selon la revendication 10 caractérisé par le fait que le(s) lipide(s) amphiphile(s) non-ionique(s) constituant les feuillets des vésicules a (ou ont) pour formule : 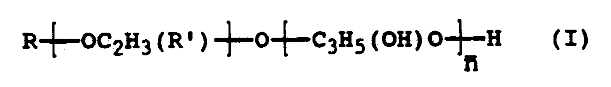 dans laquelle :
· -OC₂H₃(R')- désigne les structures suivantes, prises en mélange ou séparément : 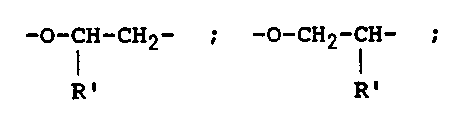 R et R' représentant chacun une chaîne aliphatique, linéaire ou ramifiée, saturée ou insaturée, contenant de 12 à 18 atomes de carbone, ou bien un reste R¹CO où R¹ est un radical aliphatique, linéaire ou ramifié, en C₁₁-C₁₇ ;
· -C₃H₅(OH)O- désigne les structures suivantes, prises en mélange ou séparément :
-CH₂-CH(OH)-CH₂-O- ; 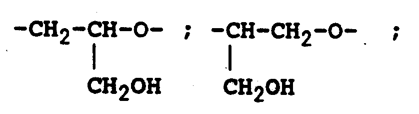 et
· n̅ est une valeur statistique moyenne comprise entre 2 et 6.

12. Composition selon l'une des revendications 10 et 11, caractérisée par le fait qu'au(x) lipide(s) amphiphile(s) non-ionique(s) est associé au moins un additif destiné à modifier la perméabilité ou la charge superficielle des vésicules et choisi dans le groupe formé par les esters phosphoriques d'alcools gras et les sels de sodium correspondants, les alcools et diols à longue chaîne, les stérols, les amines à longue chaîne et leurs dérivés ammonium quaternaires, les bis-hydroxyalkylamines, les amines grasses polyoxyéthylénées et leurs sels, les esters d'amino-alcools à longue chaîne et leurs sels et dérivés ammonium quaternaires, les alkylsulfates et les dérivés ioniques des stérols.

13. Composition selon la revendication 12, caractérisée par le fait que l'additif est un ester phosphorique d'alcool gras ou le sel de sodium correspondant.

14. Composition selon la revendication 13, caractérisée par le fait que l'ester phosphorique d'alcool gras est le dihexadécyl ou le ditétradécylphosphate ou les sels de sodium correspondants.

15. Composition selon l'une des revendications 13 ou 14, caractérisée par le fait que l'ester phosphorique d'alcool gras est associé à du cholestérol.

16. Composition selon l'une des revendications 10 à 15, caractérisée par le fait que le(s) agent(s) antifongique(s) de la famille des imidazole introduit(s) dans la phase lipidique lamellaire des vésicules représente(nt) de 0,05 à 1% en poids par rapport au poids total de la composition.

17. Composition selon l'une des revendications 10 à 16, caractérisée par le fait que la phase aqueuse D est une dispersion d'un liquide non-miscible à l'eau dans un milieu aqueux continu, gélifié ou non.

18. Composition selon l'une des revendications 10 à 17, caractérisée par le fait que la phase aqueuse D est gélifiée.

19. Composition selon l'une des revendications 17 ou 18, caractérisée par le fait que l'agent gélifiant utilisé est pris dans le groupe formé par les acides polyacryliques réticulés, les dérivés de cellulose, les copolymères acrylamide/acrylate de sodium, les copolymères vinylpyrrolidone/acétate de vinyle, les dérivés d'algues et les gommes naturelles.

20. Composition selon la revendication 17, caractérisée par le fait que le liquide non-miscible à l'eau est une huile.

21. Composition selon la revendication 20, caractérisée par le fait que l'huile est un ester d'acide gras et de polyol ou un ester d'acide gras et d'alcool ramifié de formule R²-COOR³, dans laquelle R² représente le reste d'un acide gras en C₈-C₂₀ et R³ représente une chaîne hydrocarbonée ramifiée en C₃-C₂₀.

22. Composition selon la revendication 17, caractérisée par le fait que le liquide non-miscible à l'eau est un hydrocarbure ou un polysiloxane.

23. Composition selon l'une des revendications 10 à 22, caractérisée par le fait que la phase aqueuse E encapsulée dans les vésicules et/ou la phase aqueuse D contient (ou contiennent) au moins un additif hydrosoluble pharmaceutiquement acceptable.

24. Composition selon la revendication 23, caractérisée par le fait que l'additif hydrosoluble est pris dans le groupe formé par les bactéricides, les antioxydants, les conservateurs, les agents chélatants, les opacifiants et les colorants.

25. Composition selon l'une des revendications 17 et 19 à 22, éventuellement prise en combinaison avec la revendication 23, caractérisée par le fait que le liquide non-miscible à l'eau dispersé dans la phase aqueuse D contient au moins un additif liposoluble pharmaceutiquement acceptable.

26. Composition selon l'une des revendications 17 et 19 à 22, caractérisée par le fait que les feuillets lipidiques contiennent au moins un additif liposoluble pharmaceutiquement acceptable.

27. Composition selon l'une des revendications 25 ou 26, caractérisée par le fait que l'additif liposoluble est pris dans le groupe formé par les antioxydants, les conservateurs, les émollients.

## Claims

1. Process for improving the therapeutic efficacy of fat-soluble antifungal agents belonging to the imidazole family, in which at least one of the said antifungal agents is introduced into a nonionic amphiphilic lipid phase capable of forming, by dispersion in an aqueous phase, vesicles consisting of lamellar membranes, and the said vesicles encapsulating an aqueous phase are formed in a known manner, characterized in that one (or more) lipid(s) having two fatty chains is/are chosen as nonionic amphiphilic lipid(s), and the pH of the vesicular phase is adjusted using at least one neutralizing agent to a value between 6 and 8.

2. Process according to Claim 1, characterized in that the antifungal agent(s) is/are introduced into the lipid phase in base form.

3. Process according to either of Claims 1 and 2, characterized in that the imidazole antifungal agent(s) is/are chosen from the group composed of econazole, miconazole, itraconazole, fluconazole, terconazole, clotrimazole, butoconazole, tioconazole, vibunazole, oxyconazole, sulconazole, bifonazole, fenticonazole, meticonazole, croconazole and ketoconazole.

4. Process according to one of Claims 1 to 3, characterized in that the nonionic amphiphilic lipid(s) constituting the membranes of the vesicles has/have as their formula: 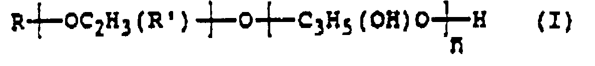 in which:
· -OC₂H₃(R')- denotes the following structures, taken mixed or separately: 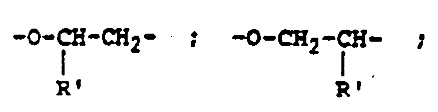 R and R' each representing a saturated or unsaturated, linear or branched aliphatic chain containing from 12 to 18 carbon atoms, or alternatively a residue R¹CO where R¹ is a linear or branched C₁₁-C₁₇ aliphatic radical,
· -C₃H₅(OH))- denotes the following structures, taken mixed or separately:
-CH₂-CH(OH)-CH₂-O- ; 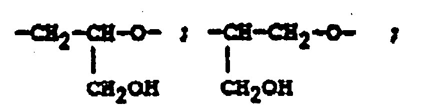 and
· n̅ is an average statistical value between 2 and 6.

5. Process according to one of Claims 1 to 4, characterized in that the nonionic amphiphilic lipid(s) is/are combined with at least one additive intended to modify the permeability or the surface charge of the vesicles and chosen from the group composed of phosphoric esters of fatty alcohols and the corresponding sodium salts, long-chain alcohols and diols, sterols, long-chain amines and their quaternary ammonium derivatives, bis-hydroxyalkylamines, polyoxyethylenated fatty amines and their salts, esters of long-chain amino alcohols and their salts and quaternary ammonium derivatives, alkyl sulphates and ionic derivatives of sterols.

6. Process according to one of Claims 1 to 5, characterized in that the antifungal agent(s) of the imidazole family which is/are contained in the lamellar lipid phase represent(s) from 1 to 12.5% by weight relative to the total lipids constituting the membranes of the vesicles.

7. Process according to one of Claims 1 to 6, characterized in that the pH of the vesicular phase is adjusted to a value between 6.5 and 7.5.

8. Process according to one of Claims 1 to 7, characterized in that the pH of the vesicular phase is adjusted using at least one base chosen from the group composed of mono-, di- or triethanolamine, diisopropanolamine, tris(hydroxymethyl)aminomethane, lysine, arginine, tetrakis(hydroxypropyl)ethylenediamine, sodium hydroxide and potassium hydroxide.

9. Process according to one of Claims 1 to 8, characterized in that the vesicles have an average diameter of between 10 nm and 5,000 nm.

10. Composition for carrying out the process as defined in one of Claims 1 to 3, characterized in that it contains an aqueous phase D in which are dispersed vesicles of nonionic amphiphilic lipid(s) having two fatty chains containing at least one antifungal agent chosen from the imidazole family.

11. Composition according to Claim 10, characterized in that the nonionic amphiphilic lipid(s) constituting the membranes of the vesicles has/have as their formula: 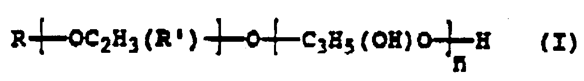 in which:
· -OC₂H₃(R')- denotes the following structures, taken mixed or separately: 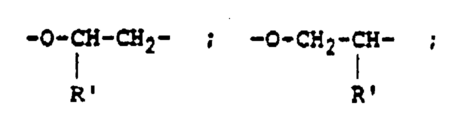 R and R' each representing a saturated or unsaturated, linear or branched aliphatic chain containing from 12 to 18 carbon atoms, or alternatively a residue R¹CO where R¹ is a linear or branched C₁₁-C₁₇ aliphatic radical;
· -C₃H₅(OH)O- denotes the following structures, taken mixed or separately:
-CH₂-CH(OH)-CH₂-O- ; 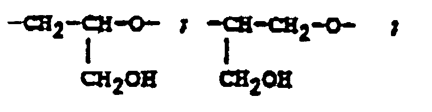 and
· n̅ is an average statistical value between 2 and 6.

12. Composition according to either of Claims 10 and 11, characterized in that the nonionic amphiphilic lipid(s) is/are combined with at least one additive intended to modify the permeability or the surface charge of the vesicles and chosen from the group composed of phosphoric esters of fatty alcohols and the corresponding sodium salts, long-chain alcohols and diols, sterols, long-chain amines and their quaternary ammonium derivatives, bis-hydroxyalkylamines, polyoxyethylenated fatty amines and their salts, esters of long-chain amino alcohols and their salts and quaternary ammonium derivatives, alkyl sulphates and ionic derivatives of sterols.

13. Composition according to Claim 12, characterized in that the additive is a phosphoric ester of a fatty alcohol or the corresponding sodium salt.

14. Composition according to Claim 13, characterized in that the phosphoric ester of a fatty alcohol is dihexadecyl or ditetradecyl phosphate or the corresponding sodium salts.

15. Composition according to either of Claims 13 and 14, characterized in that the phosphoric ester of a fatty alcohol is combined with cholesterol.

16. Composition according to one of Claims 10 to 15, characterized in that the antifungal agent(s) of the imidazole family introduced into the lamellar lipid phase of the vesicles represent(s) from 0.05 to 1% by weight relative to the total weight of the composition.

17. Composition according to one of Claims 10 to 16, characterized in that the aqueous phase D is a dispersion of a water-immiscible liquid in a continuous aqueous medium, gelled or otherwise.

18. Composition according to one of Claims 10 to 17, characterized in that the aqueous phase D is gelled.

19. Composition according to either of Claims 17 and 18, characterized in that the gelling agent used is taken from the group composed of crosslinked polyacrylic acids, cellulose derivatives, acrylamide/sodium acrylate copolymers, vinylpyrrolidone/vinyl acetate copolymers, derivatives of algae and natural gums.

20. Composition according to Claim 17, characterized in that the water-immiscible liquid is an oil.

21. Composition according to Claim 20, characterized in that the oil is an ester of a fatty acid and a polyol or an ester of a fatty acid and a branched alcohol of formula R²-COOR³, in which R² represents the residue of a C₈-C₂₀ fatty acid and R³ represents a branched C₃-C₂₀ hydrocarbon chain.

22. Composition according to Claim 17, characterized in that the water-immiscible liquid is a hydrocarbon or a polysiloxane.

23. Composition according to one of Claims 10 to 22, characterized in that the aqueous phase E encapsulated in the vesicles and/or the aqueous phase D contain(s) at least one pharmaceutically acceptable, water-soluble additive.

24. Composition according to Claim 23, characterized in that the water-soluble additive is taken from the group composed of bactericides, antioxidants, preservatives, chelating agents, opacifiers and colorants.

25. Composition according to one of Claims 17 and 19 to 22, where appropriate taken in combination with Claim 23, characterized in that the water-immiscible liquid dispersed in the aqueous phase D contains at least one pharmaceutically acceptable, fat-soluble additive.

26. Composition according to one of Claims 17 and 19 to 22, characterized in that the lipid membranes contain at least one pharmaceutically acceptable, fat-soluble additive.

27. Composition according to either of Claims 25 and 26, characterized in that the fat-soluble additive is taken from the group composed of antioxidants, preservatives and emollients.

## Patentansprüche

1. Verfahren zur Verbesserung der therapeutischen Wirksamkeit fettlöslicher antifungischer Mittel, die zur Familie der Imidazole gehören, wobei man wenigstens eines der antifungischen Mittel in eine amphiphile, nicht-ionische Lipidphase einführt, die durch Dispersion in wäßriger Phase Vesikel bilden kann, die aus lamellaren Plättchen bestehen, und wobei man in bekannter Weise die Vesikel herstellt, die eine wäßrige Phase einkapseln,
**dadurch gekennzeichnet,** daß
man als nicht-ionisches, amphiphiles Lipid (nicht-ionische, amphiphile Lipide) ein Lipid (oder mehrere Lipide) mit zwei Fettketten auswählt, und daß man den pH der Vesikelphase mit Hilfe wenigstens eines Neutralisationsmittels auf einen Wert von 6 bis 8 einstellt.

2. Verfahren gemäß Anspruch 1, dadurch gekennzeichnet, daß das antifungische Mittel (die antifungischen Mittel) in Form der Base in die Lipidphase eingeführt wird (eingeführt werden).

3. Verfahren gemäß Anspruch 1 oder 2, dadurch gekennzeichnet, daß das antifungische Imidazolmittel (die antifungischen Imidazolmittel) ausgewählt ist (ausgewählt sind) unter Econazol, Miconazol, Itraconazol, Fluconazol, Terconazol, Clotrimazol, Butoconazol, Tioconazol, Vibunazol, Oxyconazol, Sulconazol, Bifonazol, Fenticonazol, Meticonazol, Croconazol und Ketoconazol.

4. Verfahren gemäß einem der Ansprüche 1 bis 3, dadurch gekennzeichnet, daß das nicht-ionische, amphiphile Lipid (die nicht-ionischen, amphiphilen Lipide), welches (welche) die Plättchen der Vesikel bildet (bilden), folgende Formel besitzt (oder besitzen): 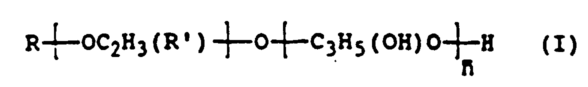 worin
· -OC₂H₃(R')- für die folgenden Strukturen, einzeln oder im Gemisch, steht: 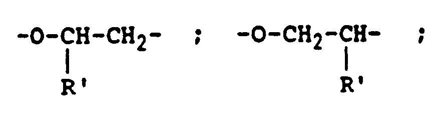 wobei R und R' jeweils für eine aliphatische, geradkettige oder verzweigte, gesättigte oder ungesättigte Kette mit 12 bis 18 Kohlenstoffatomen oder für einen R¹CO-Rest stehen, worin R¹ ein aliphatischer, geradkettiger oder verzweigter C₁₁-C₁₇-Rest ist;
· C₃H₅(OH)O- für die folgenden Strukturen, einzeln oder im Gemisch, steht:
-CH₂-CH(OH)-CH₂-O- ; 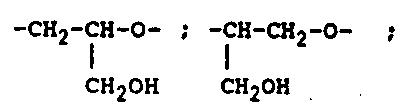 und
· n̅ ein statistischer Mittelwert von 2 bis 6 ist.

5. Verfahren gemäß einem der Ansprüche 1 bis 4, dadurch gekennzeichnet, daß mit dem (den) amphiphilen, nicht-ionischen Lipid (Lipiden) wenigstens ein Additiv assoziiert ist, das dazu bestimmt ist, die Permeabilität oder die Oberflächenladung der Vesikel zu modifizieren und welches (welche) ausgewählt ist (sind) unter Phosphorsäureestern von Fettalkoholen und den entsprechenden Natriumsalzen davon, langkettigen Alkoholen und Diolen, Sterolen, langkettigen Aminen und ihren quaternären Ammoniumderivaten, Bis-hydroxyalkylaminen, polyoxyethylenierten Fettaminen und ihren Salzen, langkettigen Aminoalkoholestern und ihren Salzen und quaternären Ammoniumderivaten, Alkylsulfaten und ionischen Sterolderivaten.

6. Verfahren gemäß einem der Ansprüche 1 bis 5, dadurch gekennzeichnet, daß das antifungische Mittel (die antifungischen Mittel) aus der Imidazolfamilie, welches (welche) in der lamellaren Lipidphase enthalten ist (sind), in einer Menge von 1 bis 12,5 Gew.-%, bezogen auf das Gesamtgewicht der die Vesikelplättchen bildenden Lipide, vorliegt (vorliegen).

7. Verfahren gemäß einem der Ansprüche 1 bis 6, dadurch gekennzeichnet, daß man den pH der Vesikelphase auf einen Wert von 6,5 bis 7,5 einstellt.

8. Verfahren gemäß einem der Ansprüche 1 bis 7, dadurch gekennzeichnet, daß man den pH der Vesikelphase mit Hilfe wenigstens einer Base einstellt, die ausgewählt wird unter Mono-, Di- oder Triethanolamin, Diisopropanolamin, Tris-hydroxymethylaminomethan, Lysin, Arginin, Tetra-hydroxypropylethylendiamin, Natriumhydroxid und Kaliumhydroxid.

9. Verfahren gemäß einem der Ansprüche 1 bis 8, dadurch gekennzeichnet, daß die Vesikel einen mittleren Durchmesser von 10 nm bis 5 000 nm besitzen.

10. Mittel zur Anwendung in einem Verfahren gemäß einem der Ansprüche 1 bis 3, dadurch gekennzeichnet, daß es eine wäßrige Phase D umfaßt, worin Vesikel aus einem nicht-ionischen, amphiphilen Lipid (nicht-ionischen, amphiphilen Lipiden) mit zwei Fettketten dispergiert sind, welche wenigstens ein antifungisches Mittel der Imidazolfamilie enthalten.

11. Mittel gemäß Anspruch 10, dadurch gekennzeichnet, daß das nicht-ionische, amphiphile Lipid (die nicht-ionischen, amphiphilen Lipide), welches (welche) die Plättchen der Vesikel bildet (bilden), folgende Formel besitzt (oder besitzen): 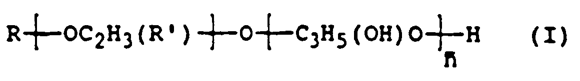 worin
· -OC₂H₃(R')- für die folgenden Strukturen, einzeln oder im Gemisch, steht: 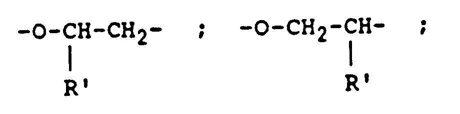 wobei R und R' jeweils für eine aliphatische, geradkettige oder verzweigte, gesättigte oder ungesättigte Kette mit 12 bis 18 Kohlenstoffatomen oder für einen R¹CO-Rest stehen, worin R¹ ein aliphatischer, geradkettiger oder verzweigter C₁₁-C₁₇-Rest ist;
· -C₃H₅(OH)O- für die folgenden Strukturen, einzeln oder im Gemisch, steht:
-CH₂-CH(OH)-CH₂-O- ; 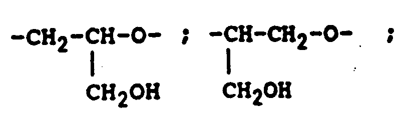 und
· n̅ ein statistischer Mittelwert von 2 bis 6 ist.

12. Mittel gemäß einem der Ansprüche 10 und 11, dadurch gekennzeichnet, daß mit dem (den) amphiphilen, nicht-ionischen Lipid (Lipiden) wenigstens ein Additiv assoziiert ist, das dazu bestimmt ist, die Permeabilität oder die Oberflächenladung der Vesikel zu modifizieren und welches (welche) ausgewählt ist (sind) unter Phosphorsäureestern von Fettalkoholen und den entsprechenden Natriumsalzen davon, langkettigen Alkoholen und Diolen, Sterolen, langkettigen Aminen und ihren quaternären Ammoniumderivaten, Bis-hydroxyalkylaminen, polyoxyethylenierten Fettaminen und ihren Salzen, langkettigen Aminoalkoholestern und ihren Salzen und quaternären Ammoniumderivaten, Alkylsulfaten und ionischen Sterolderivaten.

13. Mittel gemäß Anspruch 12, dadurch gekennzeichnet, daß das Additiv ein Phosphorsäure-Fettalkoholester oder das entsprechende Natriumsalz ist.

14. Mittel gemäß Anspruch 13, dadurch gekennzeichnet, daß es sich bei dem Phosphorsäure-Fettalkoholester um Dihexadecyl- oder Ditetradecylphosphat oder die entsprechenden Natriumsalze handelt.

15. Mittel gemäß Anspruch 13 oder 14, dadurch gekennzeichnet, daß der Phosphorsäure-Fettalkoholester mit Cholesterin assoziiert ist.

16. Mittel gemäß einem der Ansprüche 10 bis 15, dadurch gekennzeichnet, daß das antifungische Mittel (die antifungischen Mittel) der Imidazolfamilie, welches (welche) in die lamellare Lipidphase der Vesikel eingeführt ist (sind), in einer Menge von 0,05 bis 1 Gew.-%, bezogen auf das Gesamtgewicht des Mittels, vorliegt (vorliegen).

17. Mittel gemäß einem der Ansprüche 10 bis 16, dadurch gekennzeichnet, daß die wäßrige Phase D eine Dispersion einer mit Wasser nicht mischbaren Flüssigkeit in einem kontinuierlichen, wäßrigen, gelierten oder nicht-gelierten Milieu ist.

18. Mittel gemäß einem der Ansprüche 10 bis 17, dadurch gekennzeichnet, daß die wäßrige Phase D geliert ist.

19. Mittel gemäß Anspruch 17 oder 18, dadurch gekennzeichnet, daß die verwendeten, gelbildenden Mittel ausgewählt sind unter vernetzter Polyacrylsäure, Cellulosederivaten, Acrylamid/Natrium-Acrylat-Copolymeren, Vinylpyrrolidon/Vinylacetat-Copolymeren, Algenderivaten und natürlichen Gummi.

20. Mittel gemäß Anspruch 17, dadurch gekennzeichnet, daß die mit Wasser nicht mischbare Flüssigkeit ein Öl ist.

21. Mittel gemäß Anspruch 20, dadurch gekennzeichnet, daß das Öl ein Ester einer Fettsäure und eines Polyols oder ein Ester einer Fettsäure und eines verzweigten Alkohols der Formel R²-COOR³ ist, worin R² einen C₈-C₂₀-Fettsäurerest bedeutet und R³ eine verzweigte C₃-C₂₀-Kohlenwasserstoffkette darstellt.

22. Mittel gemäß Anspruch 17, dadurch gekennzeichnet, daß die mit Wasser nicht mischbare Flüssigkeit ein Kohlenwasserstoff oder ein Polysiloxan ist.

23. Mittel gemäß einem der Ansprüche 10 bis 22, dadurch gekennzeichnet, daß die wäßrige Phase E, welche die Vesikel einschließt, und/oder die wäßrige Phase D wenigstens ein wasserlösliches, pharmazeutisch akzeptables Additiv enthält (oder enthalten).

24. Mittel gemäß Anspruch 23, dadurch gekennzeichnet, daß das wasserlösliche Additiv unter Bakteriziden, Antioxidantien, Konservierungsmitteln, Chelatisierungsmitteln, opak-machenden Mitteln und Farbstoffen ausgewählt ist.

25. Mittel gemäß einem der Ansprüche 17 und 19 bis 22, gegebenenfalls in Kombination mit Anspruch 23, dadurch gekennzeichnet, daß die in der wäßrigen Phase D dispergierte, mit Wasser nicht mischbare Flüssigkeit mindestens ein fettlösliches, pharmazeutisch akzeptables Additiv enthält.

26. Mittel gemäß einem der Ansprüche 17 und 19 bis 22, dadurch gekennzeichnet, daß die Lipidplättchen wenigstens ein fettlösliches, pharmazeutisch akzeptables Additiv enthalten.

27. Mittel gemäß einem der Ansprüche 25 oder 26, dadurch gekennzeichnet, daß das fettlösliche Additiv unter Antioxidantien, Konservierungsmitteln und Emollienzien ausgewählt ist.
